(19) Europäisches Patentamt
European Patent Office
Office européen des brevets

(11) EP 0 747 365 B1

(12) **EUROPEAN PATENT SPECIFICATION**

(45) Date of publication and mention of the grant of the patent:
**11.08.2004 Bulletin 2004/33**

(51) Int Cl.[7]: **C07D 239/52**, C07D 239/30

(21) Application number: **96303928.4**

(22) Date of filing: **31.05.1996**

(54) **Process and intermediates for the manufacture of herbicidal 1-[2-(cyclopropylcarbonyl)phenyl] sulfamoyl -3-(4,6-dialkoxy-2-pyrimidinyl)urea compounds**

Verfahren und Zwischenprodukte für die Herstellung von herbizid wirkenden 1-[2-(Cyclopropylcarbonyl)phenyl]sulfamoyl-3-(4,6-dialkoxy-2-pyrimidinyl)harnstoff-Verbindungen

Procédé et produits intermédiaires pour la préparation des composés herbicides de 1-[2-(cyclopropylcarbonyl)phényl]sulfamoyl-3-(4,6-dialkoxy-2-pyrimidinyl)urée

(84) Designated Contracting States:
**AT BE CH DE DK ES FI FR GB GR IE IT LI LU MC NL PT SE**

(30) Priority: **06.06.1995 US 468101**
**06.06.1995 US 465049**

(43) Date of publication of application:
**11.12.1996 Bulletin 1996/50**

(73) Proprietor: **BASF AKTIENGESELLSCHAFT**
**67056 Ludwigshafen (DE)**

(72) Inventor: **Drabb, Thomas Walter, Jr.**
**Trenton, New Jersey 08610 (US)**

(74) Representative: **Köster, Reinhold, Dr. et al**
**BASF Aktiengesellschaft**
**Patentabteilung ZDX/P-C6**
**67056 Ludwigshafen (DE)**

(56) References cited:

**EP-A- 0 470 430**
**EP-A- 0 679 646**
**DE-A- 4 337 847**
**DE-A- 4 414 476**
**US-A- 3 939 158**
**US-A- 4 369 320**
**US-A- 4 401 816**
**US-A- 4 954 164**
**US-A- 5 009 699**
**US-A- 5 280 007**

- **D.J. BROWN ET AL.: "The chemistry of heterocyclic compounds, vol. 52: The pyrimidines" 1994 , JOHN WILEY & SONS , NEW YORK XP002013547 * pages 397-401, especially page 397, first paragraph of chapter 6.7.4.; page 400, line three from the bottom, page 401, line 6 ***

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 747 365 B1

## Description

1-{[2-(Cyclopropylcarbonyl)phenyl]sulfamoyl}-3-(4,6-dialkoxy-2-pyrimidinyl)urea compounds which are useful as crop-selective herbicidal agents are disclosed in U.S. Patent Nos. 5,009,699 and 5,280,007. In particular, 1-{[o-(cyclopropylcarbonyl)phenyl]-sulfamoyl}-3-(4,6-dimethoxy-2-pyrimidinyl)urea is being developed as a herbicide for control of a wide range of dicot and sedge weed species infesting cereals and rice. Additionally, 1-{[o-(cyclopropylcarbonyl)-phenyl]sulfamoyl}-3-(4,6-dimethoxy-2-pyrimidinyl)urea binds tightly to soil and would not be expected to pose a threat to groundwater by leaching from soil. Further, 1-{[o-(cyclopropylcarbonyl)phenyl]sulfamoyl}-3-(4,6-dimethoxy-2-pyrimidinyl)urea has been shown to be very safe for mammals and other non-target organisms.

Since 1-{[2-(cyclopropylcarbonyl)phenyl]-sulfamoyl}-3-(4,6-dialkoxy-2-pyrimidinyl)urea compounds are highly active, crop-selective herbicidal agents and are environmentally benign, there is ongoing research to discover and develop effective and efficient processes for their manufacture.

U.S. Patent No. 4,401,816 discloses sulfamoyl chlorides which are useful in the preparation of sulfamides and pyrrole sulfonamides. However, that patent does not disclose a process for the preparation of sulfamoyl urea compounds.

U.S. Patent No. 3,939,158 discloses N-(2,4-dihalo-s-triazin-6-yl)ureas and a process for their manufacture. However, the patentee does not describe a process for the preparation of sulfamoyl urea compounds.

It is therefore an object of the present invention to provide an effective and efficient process for the manufacture of 1-{[2-(cyclopropylcarbonyl)phenyl]-sulfamoyl}-3-(4,6-dialkoxy-2-pyrimidinyl)urea compounds.

It is also an object of the present invention to provide intermediate compounds which are useful in the process of the present invention.

The present invention relates to an effective and efficient, three step process for the manufacture of a 1-{[2-(cyclopropylcarbonyl)phenyl]sulfamoyl}-3-(4,6-dialkoxy-2-pyrimidinyl)urea having the structural formula I

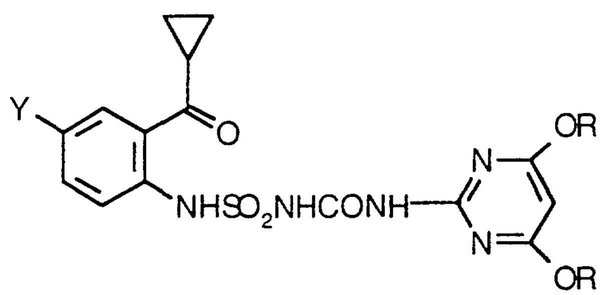

(I)

wherein

Y is hydrogen, halogen, $C_1$-$C_4$alkyl or $C_1$-$C_4$haloalkyl; and

R is $C_1$-$C_6$alkyl, which comprises reacting a 2-amino-4,6-dihalopyrimidine having the structural formula II

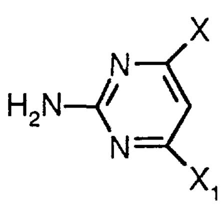

(II)

where X and $X_1$ are each independently Cl, Br or I with chlorosulfonyl isocyanate in the presence of a first solvent to obtain an intermediate compound, reacting the intermediate compound in situ with a 2-aminophenyl cyclopropyl ketone having the structural formula III

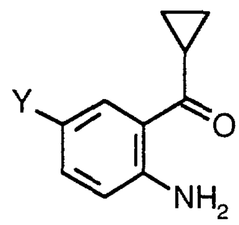

(III)

wherein Y is as described above and a base to form a 1-{[2-(cyclopropylcarbonyl)phenyl]sulfamoyl}-3-(4,6-dihalo-2-pyrimidinyl)urea having the structural formula IV

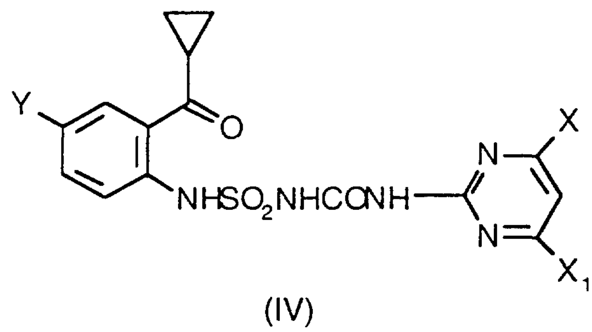

(IV)

wherein Y, X and $X_1$ are as described above, and reacting the formula IV urea with at least about two molar equivalents of an alkali metal alkoxide having the structural formula V

$$RO^- M^+$$

(V)

wherein M is an alkali metal and R is as described above in the presence of a second solvent to form the desired formula I compound.

The present invention also relates to 1-{[2-(cyclopropylcarbonyl)phenyl]sulfamoyl}-3-(4,6-dihalo-2-pyrimidinyl)ureas having the structural formula IV

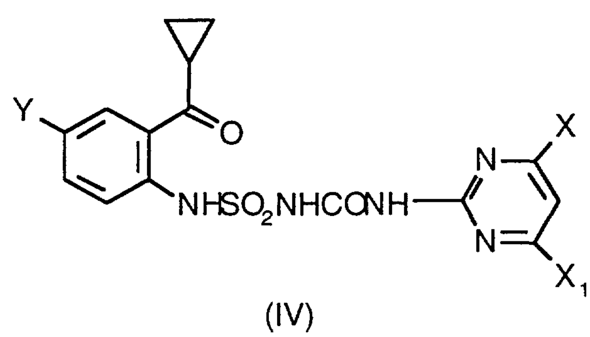

(IV)

wherein
Y is hydrogen, halogen, $C_1$ -$C_4$alkyl or $C_1$-$C_4$haloalkyl; and
X and $X_1$ are each independently Cl, Br or I. The formula IV compounds are useful as intermediate compounds in the manufacture of the crop-selective 1-{[2-(cyclopropylcarbonyl)phenyl]sulfamoyl}-3-(4,6-dialkoxy-2-pyrimidinyl)urea herbicidal agents.

The process for the preparation of the compounds of formula IV provides a further aspect of the present invention and the process of preparing the compounds of formula I from the compounds of formula IV provides a further aspect of the present invention.

Weeds cause tremendous global economic losses by reducing crop yields and lowering crop quality. In the United States alone. agronomic crops must compete with hundreds of weed species. Advantageously, it has recently been discovered that 1-{[2-(cyclopropylcarbonyl)phenyl]sulfamoyl}-3-(-4,6-dialkoxy-2-pyrimidin-yl)ureas are highly active herbicidal agents. Those compounds, especially 1-{[o-(cyclopropylcarbonyl)-phenyl]sulfamoyl}-3-(4,6-dimethoxy-2-pyrimidinyl)urea, are particularly useful for the selective control of weeds in the presence of crops.

Advantageously, it has now been found that the crop-selective herbicidal agents of formula I may be obtained in high yield and good purity by the effective and efficient, three step process of the present invention.

One of the preferred embodiments of the present invention involves reacting a formula II 2-amino-4,6-dihalopyrimidine as described above with at least about one molar equivalent of chlorosulfonyl isocyanate in the presence of a first solvent preferably at a temperature range of about -20°C to 10°C, more preferably about -5°C to 5°C, to form an intermediate compound, reacting the intermediate compound in situ with at least about one molar equivalent of a formula III 2-aminophenyl cyclopropyl ketone as described above and at least about one molar equivalent, preferably about one to three molar equivalents of a base at a temperature range of about -20°C to 30°C, more preferably about -5°C to 15°C to form a formula IV 1-{[2-(cyclopropylcarbonyl)phenyl]-sulfamoyl}-3-(4,6-dihalo-2-pyrimidinyl)urea as described above, and reacting the formula IV urea with at least about two molar equivalents, preferably about two to four molar equivalents of a formula V alkali metal alkoxide as described above in the presence of a second solvent preferably at a temperature range of about 20°C to 100°C, more preferably about 40°C to 80°C, to form a 1-{[2-(cyclopropylcarbonyl)phenyl]sulfamoyl}-3-(4,6-dialkoxy-2-pyrimidinyl)urea of formula I.

The product formula I compounds may be isolated by conventional techniques such as acidification and dilution of the reaction mixture with water followed by filtration of the formula I product or extraction of the product with a suitable solvent.

The process of the present invention is especially useful for the preparation of 1-{[o-(cyclopropylcarbonyl) phenyl]sulfamoyl}-3-(4,6-dimethoxy-2-pyrimidinyl)-urea; and 1-{[2-(cyclopropylcarbonyl)-4-fluorophenyl]-sulfamoyl}-3-(4,6-dimethoxy-2-pyrimidinyl)urea.

Surprisingly, it has been found that 1-{[2-(cyclo-propylcarbonyl)phenyl]sulfamoyl}-3-(4,6-dihalo-2-pyrimidinyl) urea compounds of formula IV are important intermediates in the manufacture of the crop-selective herbicidal agents of formula I. Formula IV compounds which are particularly suitable for use in the process of the present invention include 1-{[o-(cyclopropylcarbonyl)phenyl]sulfamoyl}-3-(4,6-dichloro-2-pyrimidinyl)urea: and 1-{[2-(cyclopropylcarbonyl)-4-fluorophenyl]sulfamoyl}-3-(4,6-dichloro-2-pyrimidinyl)urea, among others.

Alkali metals which are suitable for use in the process of the present invention include sodium, potassium and lithium with sodium and potassium being preferred. Bases for use in the invention process are tri($C_1$-$C_4$-alkyl) amines, pyridine, quinoline, with tri($C_1$-$C_4$alkyl)amines such as triethylamine being preferred.

First solvents for use in the above process are selected from the group consisting of methylene chloride and ethylene dichloride. Second solvents for use in the process of the present invention are ROH alcohols wherein R corresponds to the R group described above for formula V, and the like with ROH alcohols such as methanol being preferred.

In order to facilitate a further understanding of the invention, the following examples are presented to illustrate more specific details thereof. The invention is not to be limited thereby except as defined in the claims.

EXAMPLE 1

Preparation of 1-{[o-(Cyclopropylcarbonyl)-phenyl]sulfamoyl}-3-(4,6-dichloro-2-pyrimidinyl)urea

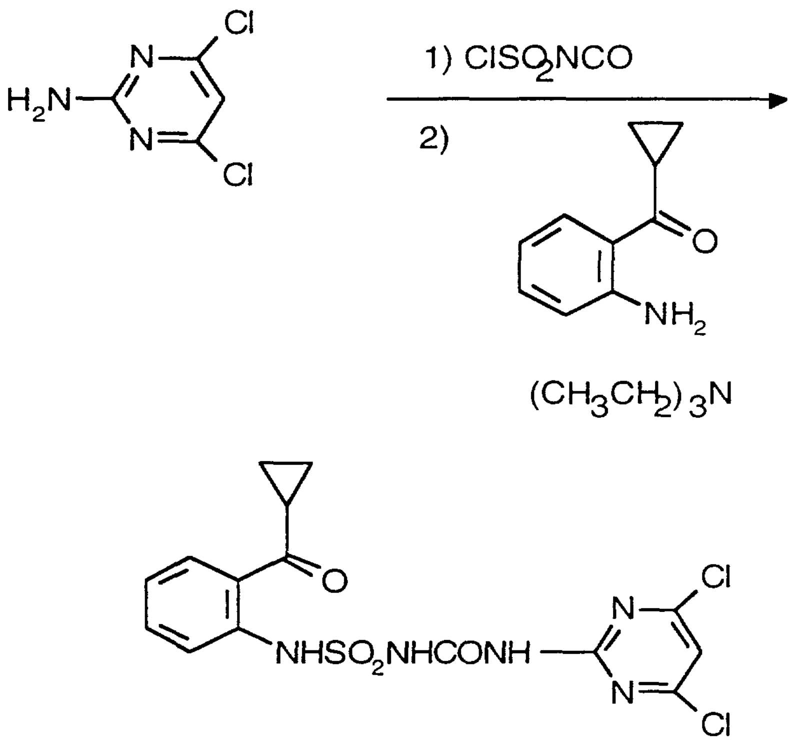


2-Amino-4,6-dichloropyrimidine (6.15 g, 0.0375 mol) is added portionwise to a solution of chloro-sulfonyl isocyanate (5.30 g, 0.0375 mol) in methylene chloride at 0°C. The resulting mixture is stirred at 0° to 2°C for two hours and a solution of o-aminophenyl cyclopropyl ketone (6.04 g, 0.0375 mol) and triethyl- amine (3.8 g, 0.0406 mol) in methylene chloride is slowly added to the mixture. The resulting solution is stirred at room temperature overnight, washed with water, dried over anhydrous magnesium sulfate and concentrated in vacuo to obtain the title product as a yellow solid (14.3 g, 75% real by HPLC analysis, 68% yield) which is used as a starting material for Example 2 without purification. A portion of the yellow solid is purified to yield the title product, mp 114-116°C.

EXAMPLE 2

Preparation of 1-{[o-(Cyclopropylcarbonyl)-phenyl]sulfamoyl}-3-(4,6-dimethoxy-2-pyrimidinyl)urea

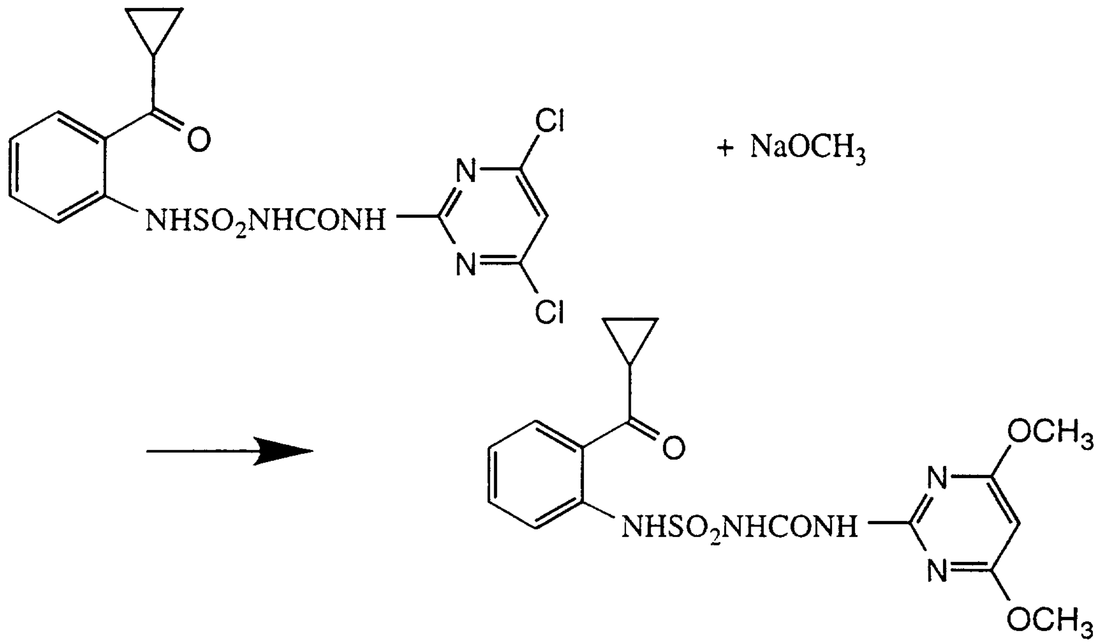


A mixture of sodium methoxide (1.08 g, 0.02 mol) in methanol is added slowly to a mixture of 1-{[o-(cyclopropyl) phenyl]sulfamoyl}-3-(4,6-dichloro-2-pyrimidinyl)urea from Example 1(4.30 g, 0.01 mol) in methanol at 60°C. The reaction mixture is heated at 60°C for two hours, cooled, treated with additional sodium methoxide (0.54 g), refluxed overnight, cooled, acidified and concentrated in vacuo to obtain a residue. The residue is slurried in methanol, filtered, washed sequentially with methanol and water, and dried in a vacuum oven at 55-60°C to give the title product as a cream colored solid (2.2 g, 98.6% real by HPLC analysis, 70% yield, mp 168.5-169.5°C).

## Claims

**1.** A process for the preparation of a compound having the structural formula (I)

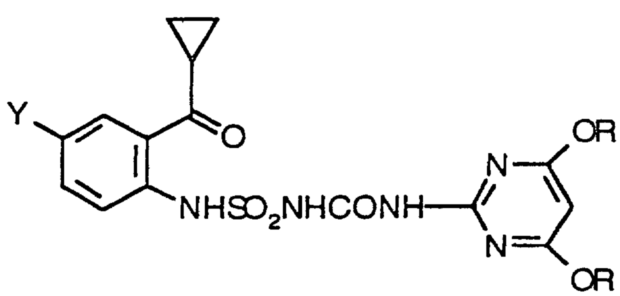

(I)

wherein
Y is hydrogen, halogen, $C_1$-$C_4$alkyl or $C_1$-$C_4$haloalkyl; and
R is $C_1$-$C_6$alkyl, which comprises reacting a 2-amino-4,6-dihalopyrimidine having the structural formula (II)

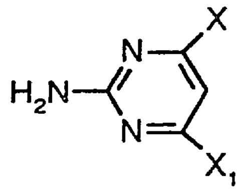

(II)

wherein X and $X_1$ are each independently Cl, Br or I with chlorosulfonyl isocyanate in the presence of a first solvent to obtain an intermediate compound, reacting the intermediate compound in situ with a 2-aminophenyl cyclopropyl ketone having the structural formula III

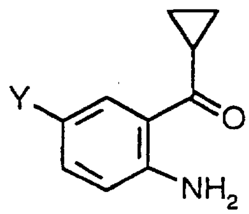

(III)

wherein Y is as described above and a base to form a 1-{[2-(cyclopropylcarbonyl)phenyl]sulfamoyl}-3-(4,6-dihalo-2-pyrimidinyl)urea having the structural formula (IV)

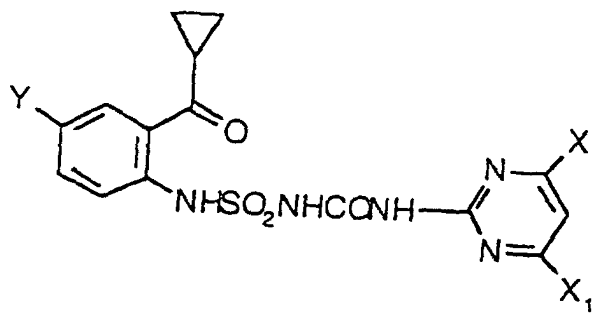

(IV)

wherein Y, X and $X_1$ are as described above, and reacting the 1-{[2-(cyclopropylcarbonyl)phenyl]-sulfamoyl}-3-(4,6-dihalo-2-pyrimidinyl)urea with at least about two molar equivalents of an alkali metal alkoxide having the structual formula (V)

$$RO^{-}M^{+} \qquad (V)$$

wherein M is an alkali metal and R is as described above in the presence of a second solvent to form the desired compound, wherein the first solvent is selected from the group consisting of methylene chloride and ethylene dichloride, the second solvent is a ROH alcohol, and the base is a tri($C_1$-$C_4$alkyl)amine, pyridine and quinoline.

**2.** A process for the preparation of a compound having the structural formula (I)

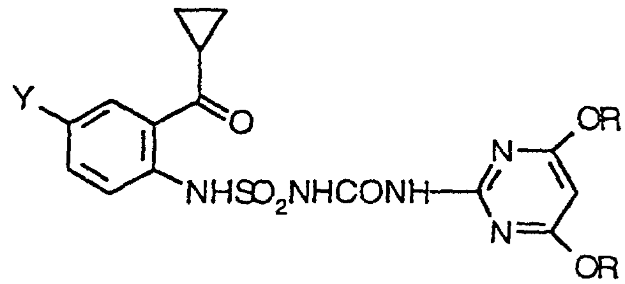

(I)

wherein
Y is hydrogen, halogen, $C_1$-$C_4$alkyl or $C_1$-$C_4$haloalkyl; and R is $C_1$-$C_6$alkyl, which comprises reacting a 1-{[2-(cyclopropylcarbonyl)phenyl]sulfamoyl}-3-(4,6-dihalo-2-pyrimidinyl)urea having the structural formula (IV)

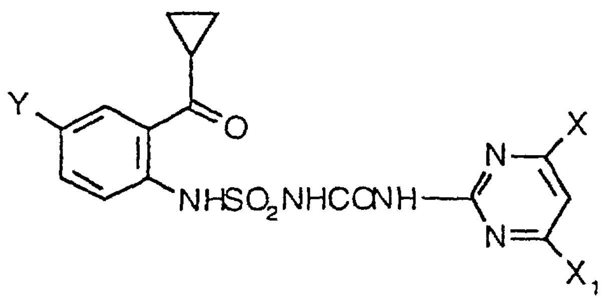

(IV)

Wherein Y is as described above and X and $X_1$ are each independently Cl, Br or I, with at least about two molar equivalents of an alkali metal alkoxide having the structual formula (V)

$$RO^-M^+ \qquad (V)$$

wherein M is an alkali metal and R is as described above in the presence of a second solvent as defined in claim 1 to form the desired compound.

**3.** A process for the preparation of a 1-{[2-(cyclopropylcarbonyl)phenyl]sulfamoyl}-3-(4,6-dihalo-2-pyrimidinyl)urea having the structural formula (IV)

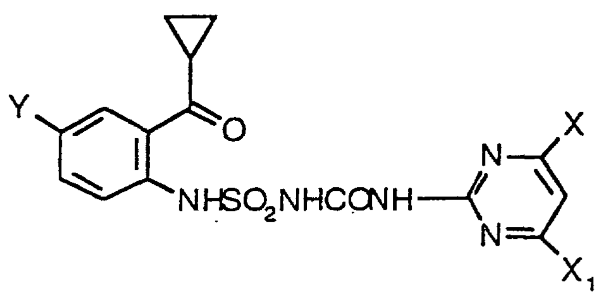

(IV)

wherein Y is as defined in claim 1 or 2 and X and $X_1$ are each independently Cl, Br or I, which comprises reacting a 2-amino-4,6-dihalopyrimidine having the structural formula (II)

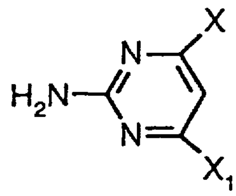

(II)

wherein X and $X_1$ are as described above, with chlorosulfonyl isocyanate in the presence of a first solvent as defined in claim 1 to obtain an intermediate compound, reacting the intermediate compound in situ with a 2-aminophenyl cyclopropyl ketone having the structural formula (III)

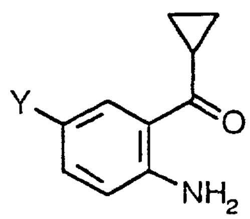

(III)

wherein Y is as described above and a base as defined in claim 1 to form the desired compound.

**4.** The process according to claims 1 or 3 wherein the base is a tri($C_1$-$C_4$alkyl)amine.

**5.** The process according to claims 1 or 4 wherein the the tri($C_1$-$C_4$alkyl)amine is triethylamine.

**6.** The process according to any of the preceding claims wherein
Y is hydrogen or F;
R is methyl;
X and $X_1$ are Cl; and
M is sodium or potassium.

**7.** The process according to any of the preceding claims wherein the 2-amino-4,6-dihalopyrimidine is reacted with the chlorosulfonyl isocyanate at a first temperature of about -20°C to 10°C, the intermediate compound is reacted with the 2-aminophenyl cyclopropyl ketone and the base at a second temperature of about -20°C to 30°C, and the 1-{[2-(cyclopropylcarbonyl)phenyl]-sufamoyl}-3-(4,6-dihalo-2-pyrimidinyl)urea is reacted with the alkali metal alkoxide at a third temperature of about 20°C to 100°C.

**8.** The process according to claim 7 wherein the first temperature is about -5°C to 5°C, the second temperature is about -5°C to 15°C, and the third temperature is about 40°C to 80°C.

**9.** A compound having the structural formula (IV)

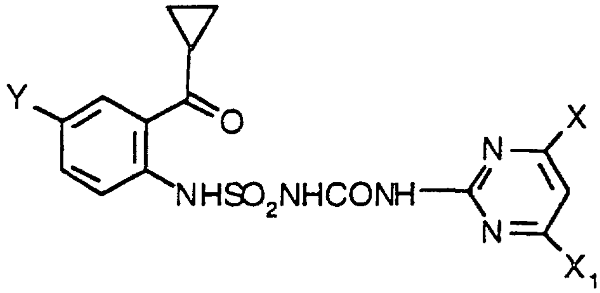 (IV)

wherein
Y is hydrogen or F; and
X and $X_1$ are each independently Cl, Br or I.

**10.** The compound according to claim 9 which is 1-{[o-(cyclopropylcarbonyl)phenyl]sulfamoyl}-3-(4,6-dichloro-2-pyrimidinyl)urea.

**11.** The compound according to claim 9 which is 1-{[2-(cyclopropylcarbonyl)-4-fluorophenyl]sulfamoyl}-3-(4,6-dichloro-2-pyrimidinyl)urea.

## Patentansprüche

**1.** Verfahren zur Herstellung einer Verbindung mit der Strukturformel (I),

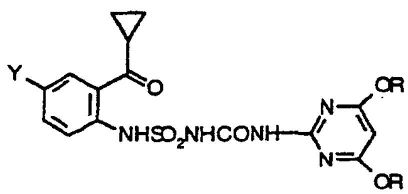 (I)

in der
Y Wasserstoff, Halogen, $C_1$-$C_4$-Alkyl oder $C_1$-$C_4$-Halogenalkyl bedeutet und
R $C_1$-$C_6$-Alkyl bedeutet,
das **dadurch gekennzeichnet ist, daß** man ein 2-Amino-4,6-dihalogenpyrimidin mit der Strukturformel (II)

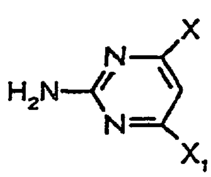 (II)

in der X und $X_1$ jeweils unabhängig Cl, Br oder I bedeuten,
mit Chlorsulfonylisocyanat in Gegenwart eines ersten Lösungsmittels zu einer Zwischenverbindung umgesetzt,
die Zwischenverbindung in situ mit einem 2-Aminophenylcyclopropylketon mit der Strukturformel (III),

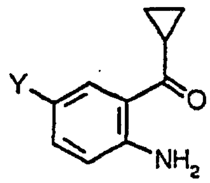

(III)

in der Y wie oben beschrieben ist,
und einer Base zu einem 1-{[2-(Cyclopropylcarbonyl)phenyl]sulfamoyl}-3-(4,6-dihalogen-2-pyrimidinyl)harnstoff mit der Strukturformel (IV),

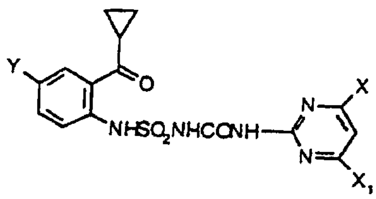

(IV)

in der Y, X und $X_1$ wie oben beschrieben sind,
umsetzt und den 1-{[2-(Cyclopropylcarbonyl)-phenyl]sulfamoyl}-3-(4,6-dihalogen-2-pyrimidinyl)-harnstoff mit mindestens ungefähr zwei Moläquivalenten eines Alkalimetallalkoholats mit der Strukturformel (V),

$$RO^{-}M^{+} \qquad (V)$$

in der M ein Alkalimetall bedeutet und R wie oben beschrieben ist,
in Gegenwart eines zweiten Lösungsmittels zu der gewünschten Verbindung umsetzt, wobei das erste Lösungsmittel aus der Gruppe Methylenchlorid und Ethylenchlorid stammt, das zweite Lösungsmittel ein ROH-Alkohol ist und die Base ein Tri($C_1$-$C_4$-alkyl)amin, Pyridin oder Chinolin ist.

**2.** Verfahren zur Herstellung einer Verbindung mit der Strukturformel (I),

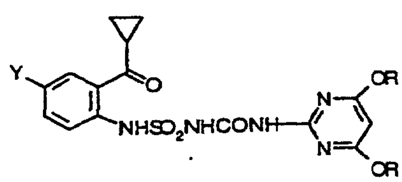

(I)

in der
Y Wasserstoff, Halogen, $C_1$-$C_4$-Alkyl oder $C_1$-$C_4$-Halogenalkyl bedeutet und
R $C_1$-$C_6$-Alkyl bedeutet,
das **dadurch gekennzeichnet ist, daß** man einen 2-{[2-(Cyclopropylcarbonyl)phenyl]sulfamoyl}-3-(4,6-dihalogen-2-pyrimidinyl)harnstoff mit der Strukturformel (IV),

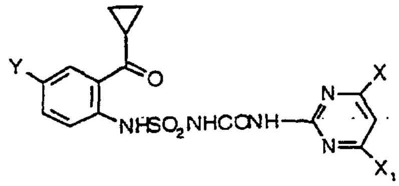

(IV)

in der Y wie oben beschrieben ist und X und $X_1$ jeweils unabhängig Cl, Br oder I bedeuten, mit mindestens ungefähr zwei Moläquivalenten eines Alkalimetallalkoholats mit der Strukturformel (V),

$$RO^-M^+ \qquad (V)$$

in der M ein Alkalimetall bedeutet und R wie oben beschrieben ist, in Gegenwart eines zweiten, wie in Anspruch 1 definierten Lösungsmittels, zu der gewünschten Verbindung umsetzt.

**3.** Verfahren zur Herstellung eines 1-{[2-(Cyclopropylcarbonyl)phenyl]sulfamoyl}-3-(4,6-dihalogen-2-pyrimidinyl) harnstoffs mit der Strukturformel (IV),

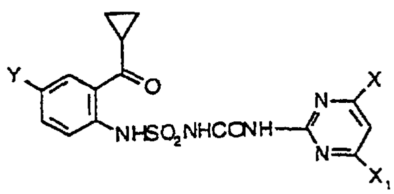

(IV)

in der Y wie in Anspruch 1 oder 2 definiert ist und X und $X_1$ jeweils unabhängig Cl, Br oder I bedeuten, das **dadurch gekennzeichnet ist, daß** man ein 2-Amino-4,6-dihalogenpyrimidin mit der Strukturformel (II)

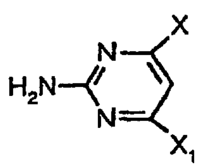

(II)

in der X und $X_1$ wie oben beschrieben sind, mit Chlorsulfonylisocyanat in Gegenwart eines ersten, wie in Anspruch 1 definierten Lösungsmittels zu einer Zwischenverbindung umsetzt, die Zwischenverbindung in situ mit einem 2-Aminophenylcyclopropylketon mit der Strukturformel (III)

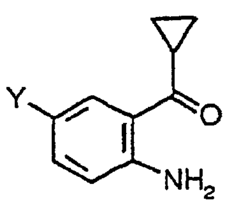

(III)

in der Y wie oben beschrieben ist, und mit einer wie in Anspruch 1 definierten Base zu der gewünschten Verbindung umsetzt.

4. Verfahren nach Anspruch 1 oder 3, wobei es sich bei der Base um ein Tri($C_1$-$C_4$-alkyl)amin handelt.

5. Verfahren nach Anspruch 1 oder 4, wobei es sich bei dem Tri($C_1$-$C_4$-alkyl)amin um Triethylamin handelt.

6. Verfahren nach einem der vorhergehenden Ansprüche, wobei
Y Wasserstoff oder F bedeutet;
R Methyl bedeutet;
X und $X_1$ Cl bedeuten; und
M Natrium oder Kalium bedeutet.

7. Verfahren nach einem der vorhergehenden Ansprüche, wobei das 2-Amino-4,6-dihalogenpyrimidin mit dem Chlorsulfonylisocyanat bei einer ersten Temperatur von ungefähr -20°C bis 10°C umgesetzt wird, die zwischenverbindung mit dem 2-Aminophenylcyclopropylketon und der Base bei einer zweiten Temperatur von ungefähr -20°C bis 30°C umgesetzt wird und der 1-{[2-(Cyclopropylcarbonyl)phenyl]-sulfamoyl}-3-(4,6-dihalogen-2-pyrimidinyl) harnstoff mit dem Alkalimetallalkoholat bei einer dritten Temperatur von ungefähr 20°C bis 100°C umgesetzt wird.

8. Verfahren nach Anspruch 7, wobei die erste Temperatur ungefähr -5°C bis 5°C, die zweite Temperatur ungefähr -5°C bis 15°C und die dritte Temperatur ungefähr 40°C bis 80°C beträgt.

9. Verbindung mit der Strukturformel (IV),

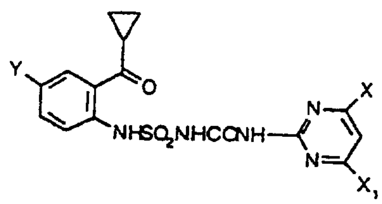

(IV)

in der
Y Wasserstoff oder F bedeutet; und
X und $X_1$ jeweils unabhängig Cl, Br oder I bedeuten.

10. Verbindung nach Anspruch 9, bei der es sich um 1-{[o-(Cyclopropylcarbonyl)phenyl]sulfamoyl}-3-(4,6-dichlor-2-pyrimidinyl)harnstoff handelt.

11. Verbindung nach Anspruch 9, bei der es sich um 1-{[2-(Cyclopropylcarbonyl)-4-fluorphenyl]-sulfamoyl}-3-(4,6-dichlor-2-pyrimidinyl)harnstoff handelt.

## Revendications

1. Procédé pour la préparation d'un composé répondant à la formule développée (1)

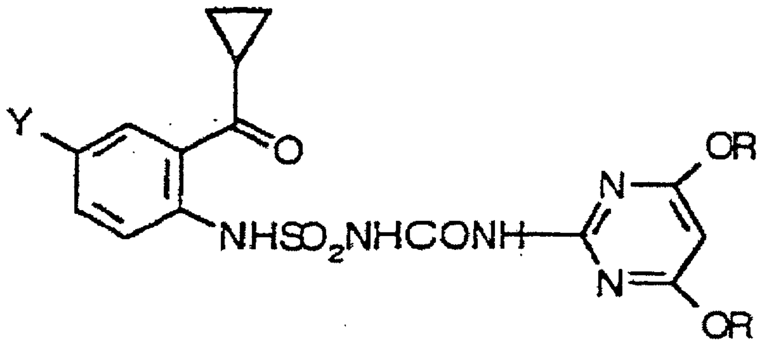

(I)

dans laquelle
Y représente un atome d'hydrogène, un atome d'halogène, un groupe alkyle en $C_1$-$C_4$ ou un groupe halogénoalkyle en $C_1$-$C_4$; et
R représente un groupe alkyle en $C_1$-$C_6$, qui comprend la mise en réaction d'une 2-amino-4,6-dihalogénopyrimidine répondant à la formule développée (II)

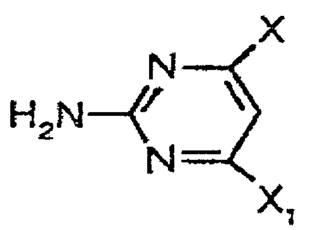

(II)

dans laquelle X et $X_1$ représentent, chacun indépendamment l'un de l'autre, un atome de chlore, un atome de brome ou un atome d'iode, avec du chlorosulfonyl isocyanate en présence d'un premier solvant pour obtenir un composé intermédiaire,
la mise en réaction du composé intermédiaire in situ avec une 2-aminophénylcyclopropylcétone répondant à la formule développée (III)

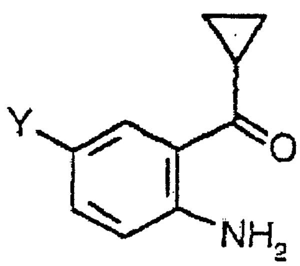

(III)

dans laquelle Y est tel que décrit ci-dessus et avec une base pour former une 1-{[2-(cyclopropylcarbonyl)phényl] sulfamoyl}-3-(4,6-dihalogéno-2-pyrimidinyl)urée répondant à la formule développée (IV)

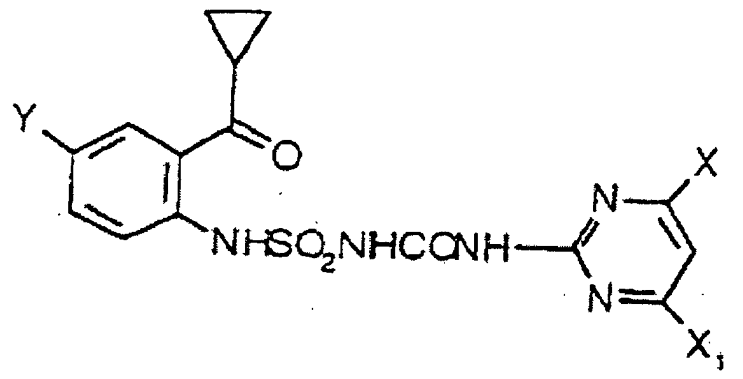

**(IV)**

dans laquelle Y, X et $X_1$ sont tels que décrits ci-dessus, et la mise en réaction de la 1-{[2-(cyctopropylcarbonyl) phényl]sulfamoyl}-3-(4,6-dihalogéno-2-pyrimidinyl)urée avec au moins environ deux équivalents molaires d'un alcoxyde de métal alcalin répondant à la formule développée (V)

$$RO^{-}M^{+}$$

(V)

dans laquelle M représente un métal alcalin et R est tel que décrit ci-dessus, en présence d'un deuxième solvant pour obtenir le composé désiré, dans lequel le premier solvant est choisi parmi le groupe constitué par le chlorure de méthylène et le dichlorure d'éthylène, le deuxième solvant est un alcool répondant à la formule ROH et la base est une tri(alkyl en $C_1$-$C_4$)amine, la pyridine et la quinoléine.

**2.** Procédé pour la préparation d'un composé répondant à la formule développée (I)

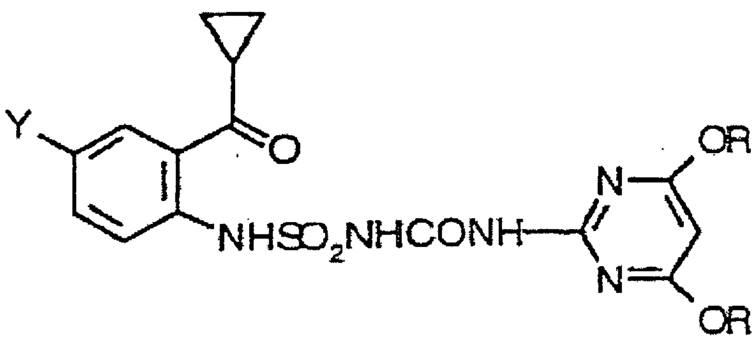

**(I)**

dans laquelle
Y représente un atome d'hydrogène, un atome d'halogène, un groupe alkyle en $C_1$-$C_4$ ou un groupe halogénoalkyle en $C_1$-$C_4$; et
R représente un groupe alkyle en $C_1$-$C_6$, qui comprend la mise en réaction d'une -1{[2-(cyclopropylcarbonyl)phényl] sulfamoyl}-3-(4,6-dihalogéno-2-pyrimidinyl)urée répondant à la formule développée (IV)

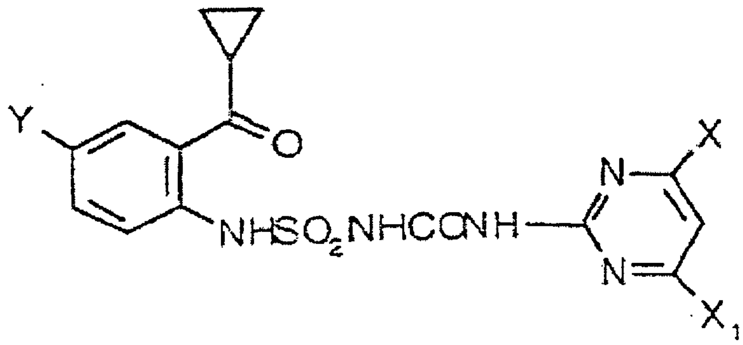

**(IV)**

dans laquelle Y est tel que décrit ci-dessus et X et $X_1$ représentent, chacun indépendamment l'un de l'autre, un atome de chlore, un atome de brome ou un atome d'iode, avec au moins environ deux équivalents molaires d'un alcoxyde de métal alcalin répondant à la formule développée (V)

$$RO^{-}M^{+}$$

(V)

dans laquelle M représente un métal alcalin et R est tel que décrit ci-dessus, en présence d'un deuxième solvant tel que défini à la revendication 1 pour obtenir le composé désiré.

3. Procédé pour la préparation d'une 1-{[2-(cyclopropylcarbonyl)-phényl]sulfamoyl}-3-(4,6-dihalogéno-2-pyrimidinyl) urée répondant à la formule développée (IV)

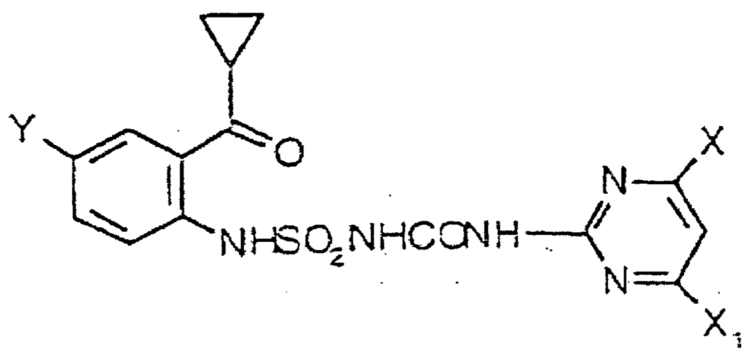

**(IV)**

dans laquelle
Y est tel que défini à la revendication 1 ou 2, et X et $X_1$ représentent, chacun indépendamment l'un de l'autre, un atome de chlore, un atome de brome ou un atome d'iode, qui comprend la mise en réaction d'une 2-amino-4,6-dihalogénopyrimidine répondant à la formule développée (II)

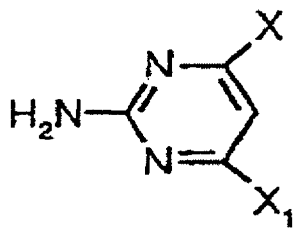

**(II)**

dans laquelle X et $X_1$ sont tels que décrits ci-dessus, avec du chlorosulfonyl isocyanate en présence d'un premier solvant tel que défini à la revendication 1 pour obtenir un composé intermédiaire,
la mise en réaction du composé intermédiaire in situ avec une 2-aminophénylcyclopropylcétone répondant à la formule développée (III)

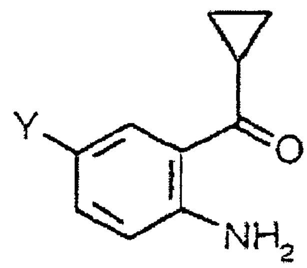

**(III)**

dans laquelle Y est tel que décrit ci-dessus et avec une base telle que définie à la revendication 1 pour obtenir le composé désiré.

**4.** Procédé selon la revendication 1 ou 3, dans lequel la base est une tri(alkyl en $C_1$-$C_4$)amine.

**5.** Procédé selon la revendication 1 ou 4, dans lequel la tri(alkyl en $C_1$-$C_4$)amine est la triéthylamine.

**6.** Procédé selon l'une quelconque des revendications précédentes,
dans lequel
Y représente un atome d'hydrogène ou un atome de fluor ;
R représente un groupe méthyle ;
X et $X_1$ représentent un atome de chlore ; et
M représente le sodium ou le potassium.

**7.** Procédé selon l'une quelconque des revendications précédentes, dans lequel on fait réagir la 2-amino-4,6-dihalogénopyrimidine avec le chloro-sulfonyl isocyanate à une première température d'environ -20 °C à 10°C, on fait réagir le composé intermédiaire avec la 2-aminophénylcyclopropylcétone et avec la base à une deuxième température d'environ -20 °C à 30 °C, et on fait réagir la 1-{[2-(cyclopropylcarbonyl)-phényl]sulfamoyl}-3-(4,6-dihaiogéno-2-pyrimidinyl)urée avec l'alcoxyde de métal alcalin à une troisième température d'environ 20 °C à 100°C.

**8.** Procédé selon la revendication 7, dans lequel la première température s'élève d'environ -5 °C à 5 °C, la deuxième température s'élève d'environ -5 °C à 15 °C et la troisième température s'élève d'environ 40 °C à 80 °C.

**9.** Composé répondant à la formule développée (IV)

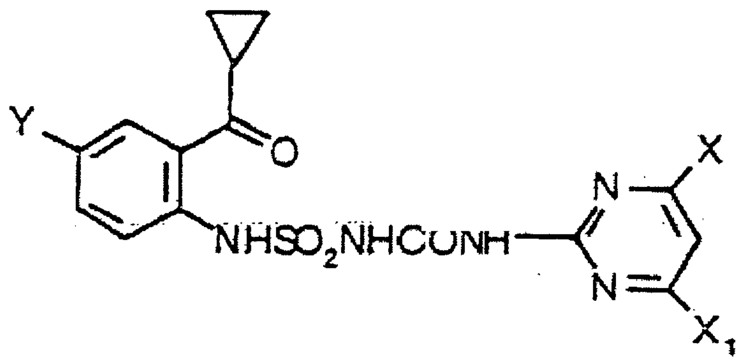

**(IV)**

dans laquelle
Y représente un atome d'hydrogène ou un atome de fluor ; et
X et $X_1$ représentent, chacun indépendamment l'un de l'autre, un atome de chlore, un atome de brome ou un atome d'iode.

**10.** Composé selon la revendication 9, à savoir la 1-{[o-(cyclopropyl-carbonyl)phényl]sulfamoyl}-3-(4,6-dichloro-2-pyrimidinyl)urée.

**11.** Composé selon la revendication 9, à savoir la 1-{[2-(cyclopropyl-carbonyl)-4-fluorophényl]sulfamoyl}-3-(4,6-dichloro-2-pyrimidinyl)urée.